# EUROPÄISCHE PATENTSCHRIFT

(11) **EP 2 404 641 B1**
(45) Veröffentlichungstag und Bekanntmachung des Hinweises auf die Patenterteilung: **27.03.2019**
(21) Anmeldenummer: 10174210.4
(22) Anmeldetag: 26.08.2010
(51) Int. Cl.: A61K 8/14, A61K 8/9789, A61K 8/44, A61K 8/49, A61Q 19/06

(54) **Kosmetische Formulierung zur Reduktion der äußeren Erscheinungsbilder von Cellulite oder Orangenhaut**
Cosmetic formula for reducing the external appearance of cellulite or dimpled skin
Formule cosmétique pour la réduction de l'apparence extérieure de la cellulite ou de la peau d'orange

(30) Priorität: 28.08.2009 DE 102009028996
(43) Veröffentlichungstag der Anmeldung: 11.01.2012
(73) Patentinhaber: Evonik Schlüchtern GmbH, 36381 Schlüchtern (DE)
(72) Erfinder: Teichmüller, Dirk, 63589, Linsengericht (DE)
(74) Vertreter: Evonik Patent Association

(56) Entgegenhaltungen:
- WO-A1-02/098436
- WO-A1-03/074011
- WO-A1-2004/096169
- WO-A2-01/19158
- US-A1- 2002 106 388
- DATABASE GNPD [Online] MINTEL; 1. Juni 2009 (2009-06-01), LPG Systems: "Body Contouring & Firming Fluid", XP002723739, Database accession no. 1124828
- DATABASE GNPD [Online] MINTEL; 1. Mai 2007 (2007-05-01), Collistar: "Pre-Sport Slimming Toning Lotion", XP002723740, Database accession no. 713100
- BOMBARDELLI E ET AL: "PHYTOSOMES IN FUNCTIONAL COSMETICS", FITOTERAPIA, IDB HOLDING, MILAN, IT, Bd. 65, Nr. 5, 1. Januar 1994 (1994-01-01) , Seiten 387-401, XP001105302, ISSN: 0367-326X
- DATABASE WPI Week 200455 Thomson Scientific, London, GB; AN 2004-566006 XP002723741, & JP 2004 217583 A (NARISU KESHOHIN KK) 5. August 2004 (2004-08-05)
- Muhammed Majeed ET AL: "Protocols of studies COLEUS FORSKOHLII EXTRACT (95% forskolin)", , 1. Juni 2013 (2013-06-01), Seiten 1-11, XP055115362, US Gefunden im Internet: URL:http://opalbond.co.uk/wp-content/uploa ds/2013/06/Forslean-writeup.pdf [gefunden am 2014-04-25]

## Beschreibung

Die vorliegende Erfindung betrifft eine kosmetische Formulierung zur effektiven Reduktion der äußeren Erscheinungsbilder von Cellulite bzw. Orangenhaut.

Cellulite (Orangenhaut) ist ein weit verbreitetes Hautproblem, das vorwiegend bei Frauen auftritt und zwar nicht selten auch bereits bei jungen Frauen und bei Frauen mit Normalgewicht. Das äußere Erscheinungsbild der Cellulite wird geprägt durch Vertiefungen oder "Dellen" in der Hautoberfläche. Betroffen ist hiervon insbesondere die Haut an den Beinen und an der Hüfte.

Zur Behandlung von Cellulite sind zahlreiche Behandlungsmethoden entwickelt worden, von denen bisher jedoch keine hinreichend zufriedenstellende Resultate liefert. Zu diesen Verfahren zählen u.a. die Lymphdrainage, die Cellulipolyse und das sogenannte "Bodywrapping". Die Lymphdrainage ist eine Spezialmassage, die den Lymphfluss wieder in Gang bringt, wodurch Wasseransammlungen im Gewebe über die Nieren abtransportiert werden und hierdurch die Orangenhaut sichtbar geglättet werden soll. Bei der Cellulipolyse werden zwei Nadeln in die Haut gestochen und dazwischen ein elektrisches Feld erzeugt, das das Unterhautfettgewebe in seine Bestandteile zerlegen soll. Beim "Bodywrapping" werden Schenkel, Hüfte oder Bauch mit Fango-, Algen- oder Schlammpackungen eingerieben und dann mit Folie umwickelt werden, wobei die unter der Folie entstehende Wärme den Lymphfluss anregen soll.

Das Dokument von der Datenbank GNPD [Online], der Firma MINTEL vom 1. Juni 2009 mit der Datenbank-Zugriffsnummer 1124828, offenbart eine Produktbeilage der Firma LPG Systems bezüglich einer "Body Contouring & Firming Fluid". Das Fluid dient zur Behandlung von Hautalterung und lokalisierter Fetteinlagerung. Das Fluid enthält laut Produktbeschreibung einen Liposomenkomplex, der eine Kombination von Koffein, L-Carnitin und Centella asiatica Extrakt und membranbildende Lipide in vesikulärer Form enthält und unter anderem den Stoff Lecithin offenbart. Laut Herstellerbeschreibung des Fluids sind 3% Liposomen enthalten, welche Lipolyse induzieren. Die Zusammensetzungen werden topisch verabreicht

Das Dokument von der Datenbank GNPD [Online], der Firma MINTEL vom 1. Mai 2007 mit der Datenbank-Zugriffsnummer 713100, offenbart eine Produktbeilage der Firma Collistar bezüglich einer "Pre-Sport Slimming Toning Lotion". Es handelt sich um ein kommerzielles Produkt zum Auftragen vor dem Sport. Die Lotion enthält, Caffeine, Coleus Forskohlii Root Extract und Liposomen (IN Cl: Lecithin, Phospholipide).

WO 02/098436 A1 offenbart in Ansprüchen 1 bis 5 Zusammensetzungen zur Behandlung von lokalen Fettansammlungen und Cellulite, die einen Komplex von Centella asiatica Triterpene (i.e. Madecassoside und Asiaticoside) in Phospholipiden, einen weiteren Komplex aus Phospholipiden und Beta-Sitosterol, sowie Coleus Forskohlii Extract enthalten. Der Extrakt ist 20%ig (Beispiel 1).

Die vorgenannten Verfahren zur Behandlung von Cellulite sind sehr aufwendig und können üblicherweise nur von Fachkräften durchgeführt werden. Es besteht daher ein Bedarf nach einer selbständig durchführbaren Behandlungsmethode, wie z.B. die tägliche Anwendung einer auf die zu behandelnden Stellen selbst aufzutragenden kosmetischen Formulierung.

Es war daher die Aufgabe der vorliegenden Erfindung eine kosmetische Formulierung bereitzustellen, die zu einer effektiven Reduktion der äußeren Erscheinungsbilder von Cellulite bzw. Orangenhaut führt als die bisher für diese Zwecke bekannten Präparate.

Diese Aufgabe wird erfindungsgemäß gelöst durch eine kosmetische Formulierung zur Reduktion der äußeren Erscheinungsbilder von Cellulite oder Orangenhaut, wobei die Formulierung
- 0,1 bis 10 Gew.-% Koffein,
- 0,1 bis 10 Gew.-% L-Carnitin,
- 0,1 bis 10 Gew.-% eines Madecassoside und/oder Asiaticoside enthaltenden *Centella asiatica*-Extraktes und
- 0,01 bis 5 Gew.-% eines Forskolin enthaltenden Extraktes einer Pflanze aus der Pflanzengattung *Coleus* oder *Plectranthus* und
- einen kosmetisch verträglichen Träger, der membranbildende Lipide in vesikulärer Form umfasst,
enthält,
wobei es sich bei dem kosmetisch verträglichen Träger um ein vesikuläres, penetrierendes Trägersystem handelt, in dem alle Wirkstoffe eingeschlossen sind,
wobei es sich bei den Wirkstoffen um Koffein, L-Carnitin, den Madecassosiden bzw. Asiaticosiden in einem *Centella asiatica*-Extrakt und um Forskolin in den Extrakten aus Pflanzen der Pflanzengattung *Coleus* oder *Plectranthus* handelt und
wobei die membranbildenden Lipide unter Phospholipiden, Ceramiden, Diacylglykosiden und Gemischen davon ausgewählt sind.

Es hat sich gezeigt, dass die primäre Ursache der Cellulite nicht darin besteht, dass die Haut an der betroffenen Stelle nicht straff genug ist. Stattdessen scheint eine vermehrte Fettaufnahme in den Adipozyten (Fettzellen) im Bindegewebe die Hauptursache zu sein. Insbesondere finden sich bei Personen, die unter Cellulite leiden, stark ausgeprägte und vergrößerte Fettgewebslobuli. Die Adipozyten und die Fettgewebslobuli, in die bei Cellulite bzw. Orangenhaut verstärkt Fett aufgenommen wird, stellen daher den Hauptangriffspunkt der Zusammensetzung gemäß der vorliegenden Erfindung bei der kosmetischen Reduktion der Erscheinungsbilder von Cellulite bzw. Orangenhaut dar.

Koffein ist ein Lipolysestimulator und regt die Mikrozirkulation der Haut an. Durch die verbesserte Durchblutung wird überflüssiges Gewebewasser leichter abtransportiert und die Entwässerung des Gewebes gefördert. Koffein aktiviert das fettspaltende und -abbauende Enzym Triacylglycerollipase und hemmt den Abbau wichtiger Substanzen, ohne die der Fettabbau nicht funktioniert. Speicherfette werden in Fettsäuren und Glycerin gespalten, und in Konsequenz nimmt das Fettzellenvolumen ab. Damit unterstützt Koffein den Fettabbau im Bindegewebe und stellt einen idealen Wirkstoff in einer Anti-Cellulite-Pflege dar.

Erfindungsgemäß wird der Wirkstoff Koffein mit den weiteren Wirkstoffen L-Carnitin, den Madecassosiden bzw. Asiaticosiden in einem *Centella asiatica*-Extrakt sowie mit Forskolin in den Extrakten aus Pflanzen der Pflanzengattung *Coleus* oder *Plectranthus* kombiniert.

L-Carnitin ist ein hauteigener, natürlicher Wirkstoff, der erfindungsgemäß deshalb in die Formulierung zur Reduktion der äußeren Erscheinungsbilder von Cellulite aufgenommen wurde, da herausgefunden wurde, dass L-Carnitin die Umwandlung von Fettkomponenten in Energie unterstützt und als eine der Schlüsselsubstanzen beim Fettverbrennungsprozess anzusehen ist. L-Carnitin ermöglicht den Transport langkettiger Fettsäuren durch die mitochondriale Membran, wo aus ihnen durch β-Oxidation Energie gewonnen wird. Insofern unterstützt das in der erfindungsgemäßen Formulierung enthaltene L-Carnitin den Fettstoffwechsel dergestalt, dass in den Adipocyten bzw. den Fettgewebslobuli bereits eingelagerte Fette schneller und in größerem Maße abgebaut werden können.

Für die weiterhin in der Formulierung enthaltenen aktiven Bestandteile der *Centella asiatica* (Tigergras), nämlich die Madecassoside und Asiaticoside, konnte gezeigt werden, dass sie stimulierende Eigenschaften bei der Synthese von Kollagen und der Bindegewebsfestigung haben. Die vorliegende Erfindung macht sich diese Erkenntnis zunutze und die in der Formulierung enthaltenen Madecassoside und Asiaticoside sollen erfindungsgemäß dazu führen, dass Kollagen und Bindegewebe wieder regenerieren, wodurch die Haut wieder weicher und elastischer werden soll. Ferner sollen die in der Formulierung enthaltenen Wirkstoffe des Tigergrases den Abtransport der Schlackestoffe aus der Fettverbrennung unterstützen und zusätzlich die Mikrozirkulation verbessern.

Bei einer Ausführungsform ist die kosmetische Formulierung gemäß der vorliegenden Erfindung dadurch gekennzeichnet, dass der verwendete *Centella asiatica*-Extrakt ein Trockenextrakt ist. Vorzugsweise handelt es sich bei dem verwendeten *Centella asiatica*-Extrakt um einen Trockenextrakt, der Madecassoside in einer Konzentration von > 10 Gew.-% enthält. Noch bevorzugter enthält der verwendete *Centella asiatica-*Trockenextrakt Madecassoside in einer Konzentration von > 30 Gew.-%. Bei einer besonders bevorzugten Ausführungsform der Erfindung beträgt der Anteil an Madecassosiden in dem verwendeten *Centella* asiatica-Trockenextrakt > 50 Gew.-%. Vorzugsweise handelt es sich bei dem verwendeten Extrakt um einen aufgereinigten Extrakt mit den vorgenannten Anteilen an Madecassosiden.

Vorzugsweise handelt es sich bei dem verwendeten *Centella asiatica*-Extrakt um einen Trockenextrakt, der Asiaticoside in einer Konzentration von > 2 Gew.-% enthält. Noch bevorzugter enthält der verwendete *Centella asiatica-Trockenextrakt* Asiaticoside in einer Konzentration von > 5 Gew.-%. Bei einer besonders bevorzugten Ausführungsform der Erfindung beträgt der Anteil an Asiaticosiden in dem verwendeten *Centella asiatica*-Trockenextrakt > 10 Gew.-%. Vorzugsweise handelt es sich bei dem verwendeten Extrakt um einen aufgereinigten Extrakt mit den vorgenannten Anteilen an Asiaticosiden.

Erfindungsgemäß sind in der Formulierung zur effektiven Reduktion der äußeren Erscheinungsbilder von Cellulite Forskolin enthaltende Extrakte der Pflanzengattung Coleus oder *Plectranthus* aufgrund zwei verschiedener Wirkungen enthalten. Zum einen konnte gezeigt werden, dass Forskolin äußerlich aufgetragen die Hautdurchblutung anregt, zum anderen soll es die Bildung des Botenstoffes cAMP, der die Fettspaltungsenzyme aktiviert, ankurbeln. Damit unterstützt und optimiert das in der erfindungsgemäßen Formulierung enthaltene Forskolin synergistisch die Anti-Cellulite-Wirkung des ebenfalls enthaltenen Koffeins.

Bei einer Ausführungsform ist die kosmetische Formulierung gemäß der vorliegenden Erfindung dadurch gekennzeichnet, dass der verwendete Extrakt einer Pflanze aus der Pflanzengattung *Coleus* oder *Plectranthus* ein Trockenextrakt ist. Vorzugsweise handelt es sich bei dem verwendeten *Coleus-* bzw. *Plectranthus*-Extrakt um einen Trockenextrakt, der Forskolin in einer Konzentration von > 10 Gew.-% enthält. Noch bevorzugter enthält der verwendete Trockenextrakt einer Pflanze aus der Pflanzengattung *Coleus* oder *Plectranthus* Forskolin in einer Konzentration von > 50 Gew.-%. Bei einer besonders bevorzugten Ausführungsform der Erfindung beträgt der Anteil an Forskolin in dem verwendeten *Coleus-* bzw. *Plectranthus*-Trockenextrakt > 80 Gew.-%. Vorzugsweise handelt es sich bei dem verwendeten Extrakt um einen aufgereinigten Extrakt mit den vorgenannten Anteilen an Forskolin.

Besonders bevorzugt stammt der verwendete Extrakt einer Pflanze aus der Pflanzengattung *Coleus* oder *Plectranthus* aus wenigstens einer Pflanze, die ausgewählt ist unter *Coleus barbatus, Coleus forskohlii* und *Plectranthus barbatus.*

Da die in der erfindungsgemäßen kosmetischen Formulierung enthaltenen Wirkstoffe zum Teil polaren Charakter besitzen, enthält die kosmetische Formulierung zusätzlich einen kosmetisch verträglichen Träger, der den Transport durch die oberen Hautschichten und durch die Zellmembranen in die Zellen optimiert, damit die Bioverfügbarkeit der wirksamen Substanzen so hoch wie möglich ist.

Gemäß der vorliegenden Erfindung umfaßt der kosmetisch verträgliche Träger membranbildende Lipide, die in vesikulärer Form, d.h. in Form von Lipidvesikeln, vorliegen.

Vorzugsweise beträgt der Anteil der membranbildenden Lipide bezogen auf das Gesamtgewicht der Formulierung 1 bis 50 Gew.-%, noch bevorzugter 2 bis 20 Gew.-%.

Bei einer Ausführungsform der Erfindung sind die membranbildenden Lipide Phospholipide. Bei einer alternativen Ausführungsform der Erfindung sind die membranbildenden Lipide Ceramide und bei einer weiteren alternativen Ausführungsform der Erfindung sind die membranbildenden Lipide Diacylglykoside. Bei einer besonderen Ausführungsform der Erfindung sind die membranbildenden Lipide Gemische von zwei oder mehreren der vorgenannten membranbildenden Lipide.

Für den Fall, dass die membranbildenden Lipide Phospholipide umfassen oder daraus bestehen, bestehen die Phospholipide vorzugsweise zu wenigstens 70 Gew.-% aus Phosphatidylcholin und besonders bevorzugt zu etwa 80 bis 90 Gew.-% aus Phosphatidylcholin.

Bestimmte Ausführungsformen der erfindungsgemäßen kosmetischen Formulierung umfassen wenigstens einen kosmetisch verträglichen Hilfsstoff. Dieser wenigstens eine kosmetisch verträgliche Hilfsstoff ist vorzugsweise unter Ethanol, 2-Propanol, Glykolen (wie z.B. 1,2-Propylenglykol, 1,3-Butylenglykol und 1,2-Pentylenglykol) und Kaliumdihydrogenphosphat ausgewählt. Bei einer Ausführungsform der Erfindung umfasst die Formulierung Gemische aus zwei oder mehr der vorgenannten Hilfsstoffe.

Vorzugsweise liegen die membranbildenden Lipide in der erfindungsgemäßen kosmetischen Formulierung in Form von Lipidvesikeln vor. Vorzugsweise weisen diese Lipidvesikel eine Teilchengröße zwischen 10 und 1000nm, bevorzugt zwischen 50 und 500nm und besonders bevorzugt zwischen 100 und 300nm auf.

Bei bevorzugten Ausführungsformen ist die erfindungsgemäße kosmetische Formulierung in eine zur dermalen Applikation geeignete kosmetische Grundlage (Gel, Creme, Lotion, Salbe, Serum etc.) eingearbeitet.

Bei der erfindungsgemäßen kosmetischen Formulierung sind alle zwingend darin enthaltenen Wirkstoffe in einem vesikulären, penetrierenden Trägersystem eingeschlossen. Vorzugsweise handelt es sich dabei um die oben beschriebenen, in Form von Lipidvesikeln vorliegenden, membranbildenden Lipide. Besonders bevorzugt liegen diese Lipidvesikel in einer zur dermalen Applikation geeigneten kosmetischen Grundlage (Gel, Creme, Lotion, Salbe, Serum etc.) eingearbeitet vor.

Für Zwecke der ursprünglichen Offenbarung wird darauf hingewiesen, dass sämtliche Merkmale, wie sie sich aus der vorliegenden Beschreibung, den Zeichnungen und den Ansprüchen für einen Fachmann erschließen, auch wenn sie konkret nur im Zusammenhang mit bestimmten weiteren Merkmalen beschrieben wurden, sowohl einzeln als auch in beliebigen Zusammenstellungen mit anderen der hier offenbarten Merkmale oder Merkmalsgruppen kombinierbar sind, soweit dies nicht ausdrücklich ausgeschlossen wurde oder technische Gegebenheiten derartige Kombinationen unmöglich oder sinnlos machen.

Auf die umfassende, explizite Darstellung sämtlicher denkbarer Merkmalskombinationen wird hier nur der Kürze und der Lesbarkeit der Beschreibung wegen verzichtet. Das unten wiedergegebene Ausführungsbeispiel 1 ist ein nicht beschränkendes Beispiel für eine mögliche Kombination von Merkmalen bzw. Merkmalsgruppen.

### Ausführungsbeispiel 1

Die nach Ausführungsbeispiel 1 hergestellte kosmetische Formulierung weist folgende Inhaltsstoffe auf:

| | |
|---|---|
| 10,00 Gew.-% | Phospholipide (Lecithin) |
| 16,00 Gew.-% | Ethanol |
| 1,00 Gew.-% | Koffein |
| 1,00 Gew.-% | L-Carnitin |
| 1,00 Gew.-% | *Centella asiatica*-Blattextrakt (Trockenextrakt mit 60 Gew.-% Madecassoside. 15 Gew.-% Asiaticoside) |
| 0,10 Gew.-% | *Coleus barbatus*-Wurzelextrakt (Trockenextrakt mit 86 Gew.-% Forskolin) |
| 0,50 Gew.-% | Kaliumdihydrogenphosphat |
| Ad 100 Gew.-% | Wasser, VE |

Um die zuvor wiedergegebene kosmetische Formulierung gemäß Ausführungsbeispiel 1 herzustellen, wurde das Lecithin in Ethanol vollständig solubilisiert. In diese Lösung wurde der *Coleus barbatus*-Extrakt unter Rühren eingearbeitet und vollständig gelöst.

Zur Herstellung der Wasserphase wurden in 95% der angegebenen Wassermenge nacheinander Koffein, L-Carnitin und der *Centella asiatica*-Blattextrakt unter Rühren eingearbeitet und vollständig gelöst.

Nach einem Homogenisationsverfahren wurde nun die Lipidphase in diese Wasserphase eingearbeitet. Abschließend wurde das Kaliumdihydrogenphosphat in der verbleibenden Menge an Wasser vollständig gelöst und diese Lösung unter Rühren zugesetzt. Der pH-Wert der dünnviskosen, gelb-braunen Lipidvesikelpräparation wurde mit 30%iger Natronlauge auf pH 6.4 angehoben.

Die Teilchengröße der nach diesem Verfahren hergestellten Lipidvesikel wurde mit Hilfe der Photonenkorrelationsspektroskopie (PCS, Zetamaster S, Malvern Instruments Ltd., UK) kontrolliert, wobei der Herstellungsprozess nach Erreichen einer mittleren Teilchengröße mit einem Durchmesser von etwa 100 - 300nm beendet wurde. Als genauer Wert wurde bei diesem Ausführungsbeispiel 1 die Teilchengröße zu 145nm bestimmt.

### Studie 1 - In vitro-Studie: Lipolyse / Reduktion der Adipozyten

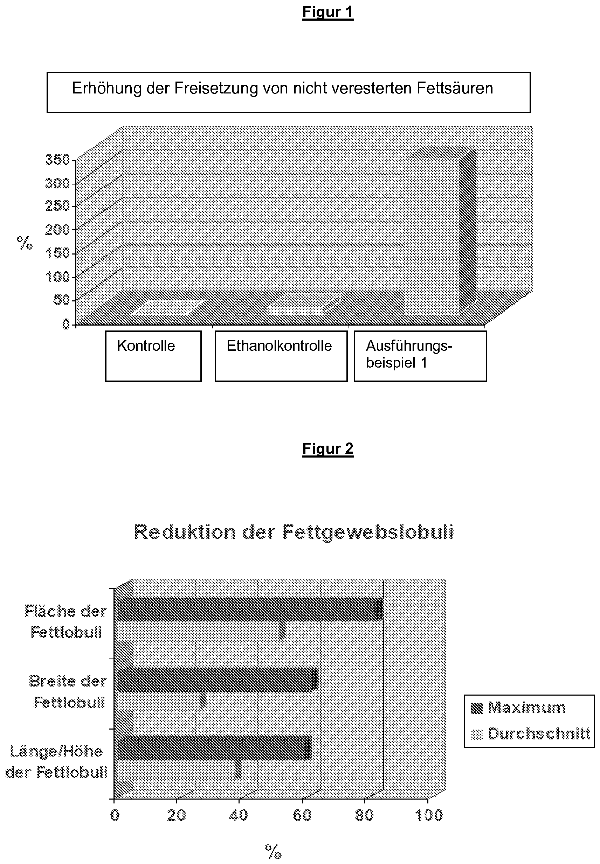

Im Rahmen einer *in vitro*-Studie wurde die erfindungsgemäße kosmetische Formulierung mit einer Zusammensetzung gemäß Ausführungsbeispiel 1 in einer Konzentration von 3 Gew.-% auf isolierte humane Adipozyten aufgebracht. Nach einer Inkubationszeit von 2 Stunden wurde die lipolytische Aktivität anhand der Menge der freigesetzen nicht veresterten Fettsäuren bestimmt. Wie in Figur 1 dargestellt ist, konnte im Ergebnis festgestellt werden, dass die erfindungsgemäße kosmetische Formulierung die Freisetzung der nicht veresterten Fettsäuren im Vergleich zu unbehandelten Adipozyten (= "Kontrolle") um >250% erhöhte. Zusätzlich wurde der Versuch auch mit einer "Ethanol-Kontrolle" (0,5 Gew.-%ige wässrige Ethanollösung) durchgeführt, um sicher zu stellen, dass der Zelltest nicht durch die Anwesenheit von Ethanol beeinträchtigt wird.

### Studie 2 - In vivo-Studie: Reduktion der Fläche der Fettgewebslobuli

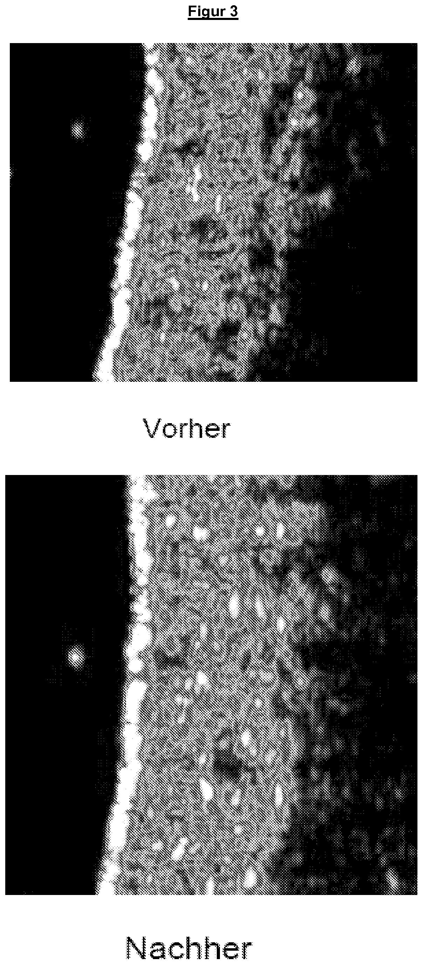

Im Rahmen einer *in vivo*-Studie an 20 weiblichen Probanden mit subjektiv starker Cellulite wurde die erfindungsgemäße Formulierung mit einer Zusammensetzung gemäß Ausführungsbeispiel 1 in einer Konzentration von 5% Gew.-% in ein angefärbtes, konserviertes und parfümiertes kosmetisches Standardcarbomergel eingearbeitet und für die Dauer von 8 Wochen jeweils morgens und abends im Bereich der Oberschenkel appliziert. Die Probanden wurden zu Beginn der Studie klinisch-dermatologisch untersucht und die Cellulite-Ausdehnung mittels Ultraschallmessung bestimmt (Dermascan C der Fa. Cortex). Nach 8 Wochen wurden die prolabierenden Fettgewebslobuli mittels Ultraschall sowohl hinsichtlich Breite, Länge/Höhe und Fläche vermessen. Wie in den Figuren 2 und 3 dargestellt ist, konnte festgestellt werden, dass sich bei allen Probanden alle drei Parameter signifikant verringert haben. So konnte beispielsweise die Fläche der prolabierenden Fettgewebslobuli um durchschnittlich >50% reduziert werden.

### Studie 3 - Ex vivo-Studie: Penetration von hydrophilen und lipophilen Fluoreszenzmarkern

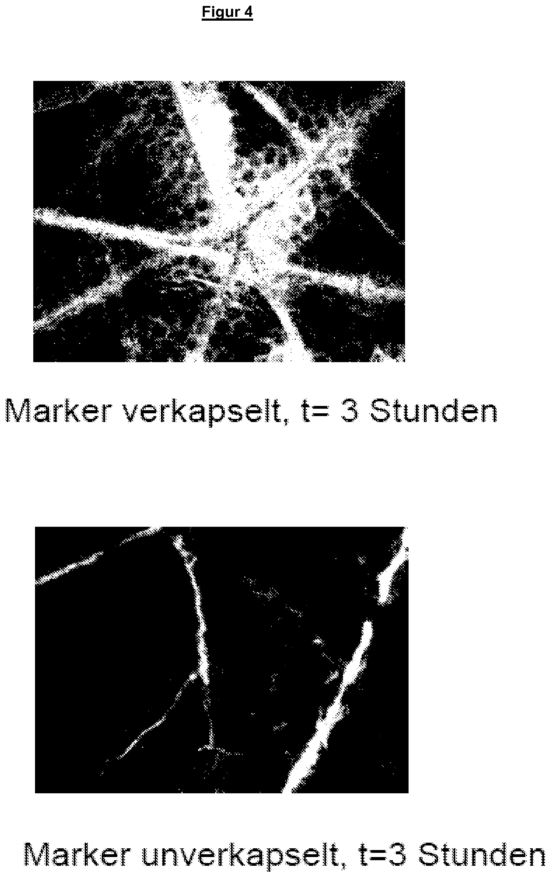

Im Verlauf einer weiteren Studie wurde die dermale Penetration des im Zusammenhang mit der erfindungsgemäßen Formulierung verwendeten Trägersystems nachgewiesen. Das Trägersystem wurde sowohl mit hydrophilen als auch mit lipophilen Fluoreszenzmarkern beladen und auf humane Hautbiopsien aufgebracht. Nach einer Inkubationszeit von 1 und 3 Stunden wurden die Biopsien gereinigt, schockgefroren und mittels Cryotom geschnitten. Die Penetration der verwendeten hydrophilen und lipophilen Fluoreszenzmarker in die Haut wurde mittels konfocaler Laserscanfluoreszenzmikroskopie (CLSM) des Hautquerschnittes visualisiert. Als Vergleich diente das Penetrationsverhalten der nicht verkapselten Fluoereszenzmarker. Wie in Figur 4 zu erkennen ist, konnte im Ergebnis festgestellt werden, dass das verwendete Trägersystem die dermale Penetration sowohl hydrophiler als auch lipophiler Wirkstoffe signifikant verstärkt.

## Patentansprüche

1. Kosmetische Formulierung zur Reduktion der äußeren Erscheinungsbilder von Cellulite oder Orangenhaut, **dadurch gekennzeichnet, dass** die Zusammensetzung
- 0,1 bis 10 Gew.-% Koffein,
- 0,1 bis 10 Gew.-% L-Carnitin,
- 0,1 bis 10 Gew.-% eines Madecassoside und/oder Asiaticoside enthaltenden *Centella asiatica*-Extraktes,
- 0,01 bis 5 Gew.-% eines Forskolin enthaltenden Extraktes einer Pflanze aus der Pflanzengattung *Coleus* oder *Plectranthus* und
- einen kosmetisch verträglichen Träger, der membranbildende Lipide in vesikulärer Form umfasst,
enthält,
wobei es sich bei dem kosmetisch verträglichen Träger um ein vesikuläres, penetrierendes Trägersystem handelt, in dem alle Wirkstoffe eingeschlossen sind,
wobei es sich bei den Wirkstoffen um Koffein, L-Carnitin, den Madecassosiden bzw. Asiaticosiden in einem *Centella asiatica*-Extrakt und um Forskolin in den Extrakten aus Pflanzen der Pflanzengattung *Coleus* oder *Plectranthus* handelt und
wobei die membranbildenden Lipide unter Phospholipiden, Ceramiden, Diacylglykosiden und Gemischen davon ausgewählt sind.

2. Kosmetische Formulierung nach Anspruch 1, **dadurch gekennzeichnet, dass** der verwendete *Centella asiatica*-Extrakt Madecassoside in einer Konzentration von > 10 Gew.-% enthält.

3. Kosmetische Formulierung nach einem der Ansprüche 1 oder 2, **dadurch gekennzeichnet, dass** der verwendete *Centella asiatica*-Extrakt Asiaticoside in einer Konzentration von > 2 Gew.-% enthält.

4. Kosmetische Formulierung nach einem der Ansprüche 1 bis 3, **dadurch gekennzeichnet, dass** der verwendete Extrakt einer Pflanze aus der Pflanzengattung *Coleus* oder *Plectranthus* aus wenigstens einer Pflanze stammt, die ausgewählt ist unter *Coleus barbatus, Coleus forskohlii* und *Plectranthus barbatus.*

5. Kosmetische Formulierung nach einem der Ansprüche 1 bis 4, **dadurch gekennzeichnet, dass** sie 1 bis 50 Gew.-% membranbildende Lipide umfasst.

6. Kosmetische Formulierung nach einem der Ansprüche 1 bis 5, **dadurch gekennzeichnet, dass** als membranbildende Lipide enthaltene Phospholipide wenigstens 70 Gew.-% Phosphatidylcholin enthalten.

7. Kosmetische Formulierung nach einem der Ansprüche 1 bis 6, **dadurch gekennzeichnet, dass** sie zusätzlich wenigstens einen kosmetisch verträglichen Hilfsstoff umfasst, der unter Ethanol, 2-Propanol, Glykolen, wie 1,2-Propylenglykol, 1,3-Butylenglykol und 1,2-Pentylenglykol, und Kaliumdihydrogenphosphat ausgewählt ist.

8. Kosmetische Formulierung nach einem der Ansprüche 1 bis 7, **dadurch gekennzeichnet, dass** die Lipidvesikel in einer Teilchengröße zwischen 10 und 1000nm vorliegen.

## Claims

1. Cosmetic formulation for the reduction of the outer appearances of cellulite or orange peel syndrome, **characterized in that** the composition contains
- from 0.1 to 10% by weight of caffeine,
- from 0.1 to 10% by weight of L-carnitine,
- from 0.1 to 10% by weight of a *Centella asiatica* extract containing madecassosides and/or asiaticosides,
- from 0.01 to 5% by weight of a forskolin-containing extract of a plant from the plant genus *Coleus* or *Plectranthus* and
- a cosmetically compatible carrier comprising membrane-forming lipids in vesicular form,
wherein the cosmetically compatible carrier is a vesicular, penetrating carrier system in which all the active ingredients are enclosed,
wherein the active ingredients are caffeine, L-carnitine, the madecassosides and/or asiaticosides in a *Centella asiatica* extract, and forskolin in the extracts from plants of the plant genus *Coleus* or *Plectranthus* and
wherein the membrane-forming lipids are selected from phospholipids, ceramides, diacyl glycosides and mixtures thereof.

2. Cosmetic formulation according to Claim 1, **characterized in that** the *Centella asiatica* extract used contains madecassosides in a concentration of > 10% by weight.

3. Cosmetic formulation according to either of Claims 1 and 2, **characterized in that** the *Centella asiatica* extract used contains asiaticosides in a concentration of > 2% by weight.

4. Cosmetic formulation according to any of Claims 1 to 3, **characterized in that** the extract used of a plant from the plant genus *Coleus* or *Plectranthus* originates from at least one plant selected from *Coleus barbatus, Coleus forskohlii* and *Plectranthus barbatus.*

5. Cosmetic formulation according to any of Claims 1 to 4, **characterized in that** it comprises from 1 to 50% by weight of membrane-forming lipids.

6. Cosmetic formulation according to any of Claims 1 to 5, **characterized in that** phospholipids present as membrane-forming lipids contain at least 70% by weight of phosphatidylcholine.

7. Cosmetic formulation according to any of Claims 1 to 6, **characterized in that** it additionally comprises at least one cosmetically compatible excipient selected from ethanol, 2-propanol, glycols, such as 1,2-propylene glycol, 1,3-butylene glycol and 1,2-pentylene glycol, and potassium dihydrogenphosphate.

8. Cosmetic formulation according to any of Claims 1 to 7, **characterized in that** the lipid vesicles are present in a particle size of between 10 and 1000 nm.

## Revendications

1. Formulation cosmétique pour la réduction de l'apparence extérieure de la cellulite ou de la peau d'orange, **caractérisée en ce que** la composition contient :
- 0,1 à 10 % en poids de caféine,
- 0,1 à 10 % en poids de L-carnitine,
- 0,1 à 10 % en poids d'un extrait de *Centella asiatica* contenant des madécassosides et/ou des asiaticosides,
- 0,01 à 5 % en poids d'un extrait d'une plante de l'espèce végétale *Coleus* ou *Plectranthus* contenant de la forskoline, et
- un vecteur cosmétiquement acceptable, qui comprend des lipides formant des membranes sous forme vésiculaire, le vecteur cosmétiquement acceptable étant un système vecteur vésiculaire pénétrant, dans lequel tous les agents actifs sont inclus,
les agents actifs étant la caféine, la L-carnitine, les madécassosides ou les asiaticosides dans un extrait de *Centella asiatica,* et la forskoline dans les extraits de plantes de l'espèce végétale *Coleus* ou *Plectranthus,* et les lipides formant des membranes étant choisis parmi les phospholipides, les céramides, les diacylglycosides et leurs mélanges.

2. Formulation cosmétique selon la revendication 1, **caractérisée en ce que** l'extrait de *Centella asiatica* utilisé contient des madécassosides en une concentration > 10 % en poids.

3. Formulation cosmétique selon l'une quelconque des revendications 1 ou 2, **caractérisée en ce que** l'extrait de *Centella asiatica* utilisé contient des asiaticosides en une concentration > 2 % en poids.

4. Formulation cosmétique selon l'une quelconque des revendications 1 à 3, **caractérisée en ce que** l'extrait d'une plante de l'espèce végétale *Coleus* ou *Plectranthus* utilisé provient d'au moins une plante, qui est choisie parmi *Coleus barbatus, Coleus forskohlii* et *Plectranthus barbatus.*

5. Formulation cosmétique selon l'une quelconque des revendications 1 à 4, **caractérisée en ce qu'**elle comprend 1 à 50 % en poids de lipides formant des membranes.

6. Formulation cosmétique selon l'une quelconque des revendications 1 à 5, **caractérisée en ce que** les phospholipides contenus en tant que lipides formant des membranes contiennent au moins 70 % en poids de phosphatidylcholine.

7. Formulation cosmétique selon l'une quelconque des revendications 1 à 6, **caractérisée en ce qu'**elle comprend en outre au moins un adjuvant cosmétiquement acceptable, qui est choisi parmi l'éthanol, le 2-propanol, les glycols, tels que le 1,2-propylène-glycol, le 1,3-butylène-glycol et le 1,2-pentylène-glycol, et le dihydrogénophosphate de potassium.

8. Formulation cosmétique selon l'une quelconque des revendications 1 à 7, **caractérisée en ce que** les vésicules lipidiques se présentent en une taille de particule comprise entre 10 et 1 000 nm.
